**Europäisches Patentamt**

**European Patent Office**

**Office européen des brevets**

(19)

(11) Veröffentlichungsnummer: **0 242 505**
B1

# EUROPÄISCHE PATENTSCHRIFT

(45) Veröffentlichungstag der Patentschrift:
07.11.90

(21) Anmeldenummer: 87100885.0

(22) Anmeldetag: 23.01.87

(51) Int. Cl.⁵: **C07D 201/08**

(54) Verfahren zur Herstellung von Caprolactam.

(30) Priorität: 28.01.86 DE 3602377

(43) Veröffentlichungstag der Anmeldung:
28.10.87 Patentblatt 87/44

(45) Bekanntmachung des Hinweises auf die Patenterteilung:
07.11.90 Patentblatt 90/45

(84) Benannte Vertragsstaaten:
BE CH DE FR GB IT LI NL

(56) Entgegenhaltungen:
EP-A- 0 075 372
DE-A- 2 535 689
DE-C- 952 442
GB-A- 1 191 539
US-A- 3 485 821

(73) Patentinhaber: BASF Aktiengesellschaft,
Carl-Bosch-Strasse 38, D-6700 Ludwigshafen(DE)

(72) Erfinder: Vagt, Uwe, Dr., Paul-Neumann-Strasse 44,
D-6720 Speyer(DE)
Erfinder: Fischer, Rolf, Dr., Bergstrasse 98,
D-6900 Heidelberg(DE)
Erfinder: Merger, Franz, Dr., Max-Slevogt-Strasse 25,
D-6710 Frankenthal(DE)
Erfinder: Hutmacher, Hans-Martin, Dr.,
Rüdigerstrasse 70, D-6700 Ludwigshafen(DE)

## Beschreibung

Gegenstand der Erfindung ist ein Verfahren zur Herstellung von Caprolactam aus 5-Formylvaleriansäureestern.

Nach einem in Journ. of the Amer. Chem. Soc., Band 64 (1942), Seite 1416 ff beschriebenen Verfahren erhält man 5-Formylvaleriansäure durch Umsetzung von 2-Hydroxicyclohexanon mit Bleitetraacetat in Eisessig. Dieses Verfahren hat den Nachteil, daß es aufwendige Ausgangsstoffe benötigt und nur in ungenügenden Ausbeuten verläuft. Zudem ist es durch den Zwangsanfall von Bleiverbindungen, die entsorgt werden müssen, belastet. Nach einem anderen in Chem. Berichte, Band 72 (1939), Seiten 1194 ff beschriebenen Verfahren erhält man 5-Formylvaleriansäure durch Umsetzen von Cyclohexanon mit alkalischem Wasserstoffperoxid. Hierbei erzielt man jedoch nur geringe Selektivitäten und benötigt lange Reaktionszeiten. Nach einem weiteren, in der EP-Anmeldung 91 091 beschriebenen Verfahren stellt man 5-Formylvaleriansäure durch Oxidation von Cyclohexanon mit molekularem Sauerstoff in Gegenwart von homogenen Katalysatoren wie z.B. Eisen(III)-chlorid und Thioharnstoff her. Nach diesem Verfahren erhält man jedoch ausreichende Selektivitäten nur bei Umsätzen von weniger als 50%. Darüber hinaus müssen die homogenen gelösten Katalysatoren aus dem Reaktionsgemisch abgetrennt werden, was aufwendig ist.

Aus der japanischen Patentveröffentlichung 21 138/66 ist auch schon ein Verfahren bekannt, bei dem man 5-Formylvaleriansäure, Ammoniak und Wasser als Verdünnungsmittel unter Mitverwendung von Wasserstoff und Hydrierkatalysatoren bei Temperaturen über 100°C zu Caprolactam umsetzt. Die Caprolactam-Ausbeuten betragen jedoch nur 20–40%. Es ist weiterhin bekannt, Caprolactam dadurch herzustellen, daß man Gemische aus 6-Hydroxicapronsäure, Adipinsäure, 5-Formylvaleriansäure und anderen Carbonsäuren zunächst bei 200–300°C in Gegenwart von Ammoniak und Hydrierkatalysatoren mit Wasserstoff behandelt, dann abkühlt, Ammoniumsalze der 5-Cyanvaleriansäure und des Adipinsäuremonoamids abtrennt, Ammoniak und Wasser erneut hinzufügt und das Reaktionsgemisch auf 300–400°C (Jap. Patentschrift 31 538 (1971). Das Verfahren erscheint wenig vorteilhaft, da es mit aufwendig vielen Schritten und schlechten Selektivitäten arbeitet. Es wurde auch beschrieben, gasförmige 5-Formylvaleriansäure (2 Vol-%) bei 280°C und Normaldruck mit Ammoniak (8 Vol-%), Wasserstoff (50 Vol-%) und Wasserdampf (40 Vol-%) in Gegenwart von Kupferoxid, Chromoxid und Manganoxid zu Caprolactam umzusetzen (GB 1 191 539). Die relativ geringe Raum-Zeit-Ausbeute und die aufgrund der in den Beispielen genannten, sehr kurzen Versuchszeiten (6 h) Probleme mit Katalysatordesaktivierung lassen das Verfahren unwirtschaftlich erscheinen.

Schließlich ist bekannt, 6-Aminocapronsäure, gelöst in Wasser (US 3 485 821), bei Temperaturen von 150 bis 350°C bzw. 170 bis 200°C zu Caprolactam zu cyclisieren.

Aus der DE-C 952 442 ist ein Verfahren bekannt, bei dem man durch hydrierende Aminierung von 5-Formylvaleriansäureestern in zwei Stufen Caprolactam neben Aminocapronsäureester erhält.

In der DE-A 2 535 689 wird ein Verfahren zur Herstellung von Caprolactam durch Cyclisieren von 6-Aminocapronsäure in Lösungsmitteln, die mindestens 60 Vol-% Ethanol oder Methanol enthalten, beschrieben.

Es war die technische Aufgabe gestellt, ein Verfahren zur Herstellung von Caprolactam aus 5-Formylvaleriansäureestern zur Verfügung zu stellen, das mit hohen Ausbeuten verläuft und bei dem wenig Nebenprodukte anfallen.

Diese Aufgabe wird gelöst in einem Verfahren zur Herstellung von ε-Caprolactam, dadurch gekennzeichnet, daß man

a) 5-Formylvaleriansäure-$C_1$ bis $C_4$-Alkylester mit 1 bis 200 Mol Wasser je Mol 5-Formylvaleriansäure-$C_1$ bis $C_4$-Alkylester in Gegenwart von stark sauren Kationenaustauschern bei einer Temperatur von 50 bis 120°C umsetzt,

b) die so erhaltene 5-Formylvaleriansäure in Form einer wäßrigen Lösung mit 2 bis 50 Mol Ammoniak und 1 bis 10 Mol Wasserstoff je Mol 5-Formylvaleriansäure in Gegenwart von Kobalt-, Nickel- oder Edelmetallkatalysatoren der VIII Gruppe bei einer Temperatur von 50 bis 150°C und unter einem Druck von 10 bis 400 bar umsetzt, und

c) die hierbei entstandene Lösung von 6-Aminocapronsäure nach Abtrennung des Ammoniaks auf Temperaturen von 150 bis 370°C, insbesondere 250 bis 330°C erhitzt und das gebildete Caprolactam abtrennt.

Das neue Verfahren hat den Vorteil, daß es auf einfache Weise gelingt, aus 5-Formylvaleriansäureestern in hohen Ausbeuten Caprolactam herzustellen. Darüber hinaus hat das neue Verfahren den Vorteil, daß es in kurzer Zeit verläuft und wenige Nebenprodukte anfallen. Ferner hat das neue Verfahren den Vorteil, daß leicht abtrennbare Katalysatoren Verwendung finden. Das neue Verfahren ist insofern bemerkenswert, als zu erwarten war, daß 5-Formylvaleriansäure in saurem Medium inter- oder intramolekulare Aldolkondensationen (Bildung von fünfgliedrigen Ringen) eingehen würde. So ist z.B. aus Houben-Weyl, Methoden der organischen Chemie, Band VII/1, Seite 87 bekannt, daß Adipindialdehyd in saurem Medium eine intramolekulare Aldolkondensation unter Bildung von Cyclopentenaldehyd eingeht.

Ausgangsstoffe sind 5-Formylvaleriansäure-$C_1$- bis $C_4$-Alkylester, beispielsweise 5-Formylvaleriansäuremethylester, 5-Formylvaleriansäureethylester, 5-Formylvaleriansäure-n-propylester, 5-Formylvaleriansäure-i-propylester, 5-Fomylvaleriansäure-n-butylester. Besondere technische Bedeutung hat 5-Formylvaleriansäuremethylester erlangt. 5-Formylvaleriansäureester sind nach dem in der EP-Anmeldung 31100 beschriebenen Verfahren durch Hydroformylierung von 4-Pentensäureestern leicht zugänglich.

Man wendet je Mol 5-Formylvaleriansäureester 1 bis 200 Mol, insbesondere 50 bis 150 Mol Wasser an. Darüber hinaus können unter den Reaktionsbedingungen flüssige inerte Lösungsmittel mitverwendet werden. Geeignete Lösungsmittel sind beispielsweise Kohlenwasserstoffe wie Cyclohexan oder Toluol, Halogenkohlenwasserstoffe wie Dichlormethan, Tetrachlormethan, Ether wie Dioxan oder Diglyme. Falls Lösungsmittel mitverwendet werden, setzt man 5-Formylvaleriansäureester als 1 bis 90 gew.-%ige Lösung, insbesondere 5 bis 20 gew.-%ige Lösung ein.

Die Umsetzung wird bei einer Temperatur von 50 bis 120°C durchgeführt. Im allgemeinen führt man die Umsetzung unter Atmosphärendruck durch. Es ist jedoch auch möglich, schwach verminderten Druck oder schwach erhöhten Druck, z.B. bis zu 20 bar anzuwenden.

Die Umsetzung wird unter Mitverwendung von stark sauren Kationenaustauschern durchgeführt. Solche stark sauren Kationenaustauscher sind beispielsweise aufgebaut aus vernetztem Polystyrol mit Sulfonsäuregruppen, Phenolharzen mit Sulfonsäuregruppen oder sauren Zeolithen.

Das Verfahren kann chargenweise oder vorteilhaft kontinuierlich, z.B. in einer Rührkesselkaskade durchgeführt werden. Hierbei ist es zweckmäßig, den bei der Hydrolyse anfallenden Alkohol fortlaufend durch Destillation aus dem Reaktionsgemisch abzutrennen. Bei der Verwendung von stark sauren Kationenaustauschern ist es vorteilhaft, die Umsetzung so durchzuführen, daß man die stark sauren Kationenaustauscher, z.B. in einem Rohrreaktor fest anordnet und das Reaktionsgemisch in Rieselfahrweise darüber leitet. In einer besonders vorteilhaften Ausführungsform wird das Reaktionsgemisch zunächst in Rieselfahrweise durch eine erste Reaktionszone mit fest angeordneten stark sauren Kationenaustauschern geleitet und dann in einer zweiten Reaktionszone über fest angeordnete stark saure Kationenaustauscher im Kreis geführt und das Reaktionsgemisch in dem Maße entnommen, wie es der ersten Zone zugeführt wird. Vorteilhaft wird aus dem so erhaltenen Umsetzungsgemsich nicht umgesetzter 5-Formylvaleriansäureester, z.B. mit unter Reaktionsbedingungen inerten und flüssigen Kohlenwasserstoffen wie Cyclohexan, extrahiert und zweckmäßig wieder verwendet.

Nach einer anderen vorteilhaften Arbeitsweise leitet man 5-Formylvaleriansäureester und Wasser im Überschuß durch eine mit stark sauren Kationenaustauschern beschickte Kolonne, destilliert am oberen Ende Alkohole ab und entnimmt am unteren Ende eine wäßrige Lösung von 5-Formylvaleriansäure.

Vorteilhaft verwendet man die wäßrige Lösung der 5-Formylvaleriansäure, die beispielsweise einen Gehalt von 3 bis 15 Gew.-% hat, direkt für die Herstellung von 6-Aminocapronsäure. In einer zweiten Stufe (Stufe b) wird dann die so erhaltene 5-Formylvaleriansäure mit überschüssigem Ammoniak und Wasserstoff in Gegenwart von Hydrierkatalysatoren und Lösungsmitteln bei einer Temperatur von 50 bis 150°C unter erhöhtem Druck umgesetzt.

5-Formylvaleriansäure wird als wäßrige Lösung im allgemeinen als 1 bis 50 gew.-%ige, vorzugsweise 2 bis 35 gew.-%ige, insbesondere 5 bis 25 gew.-%ige Lösung verwendet. Die Lösungen enthalten überwiegend Monomere und wenig niedermolekulare oligomere 5-Formylvaleriansäure. Insbesondere hat es sich bewährt, wenn man wäßrige Lösungen von 5-Formylvaleriansäure, wie sie in der Stufe a anfallen, verwendet.

Man wendet je Mol 5-Formylvaleriansäure 2 bis 50 Mol, insbesondere 4 bis 30 Mol, Ammoniak an.

Die Umsetzung führt man bei einer Temperatur von 50 bis 150°C, vorteilhaft 70 bis 130°C durch. Ferner wendet man einen Druck von 10 bis 400 bar, vorteilhaft von 20 bis 300 bar, insbesondere 50 bis 250 bar an. Wasserstoff wird in Mengenverhältnissen von 1 bis 10 Mol pro Mol 5-Formylvaleriansäure eingesetzt. Vorzugsweise verwendet man Wasserstoff im Überschuß, z.B. 2 bis 5 Mol 5-Formylvaleriansäure.

Als Hydrierkatalysatoren verwendet man Nickelkatalysatoren, Cobaltkatalysatoren oder Edelmetallkatalysatoren der 8. Gruppe des periodischen Systems, wie Ruthenium, Platin oder Palladium. Die Katalysatoren können aktivierende Zusätze wie Zirkon, Mangan, Kupfer oder Chrom enthalten. Die Katalysatoren können als Vollkatalysatoren oder auf Trägern wie Aluminiumoxid, Kieselgel, Tonerde oder Aktivkohle niedergeschlagen werden. Geeignet sind auch Skelettkatalysatoren.

Geeignete Katalysatoren enthalten z.B. 80 bis 100 Gew.-% Cobalt und/oder Nickel, bezogen auf den Metallgehalt des Katalysators. Neben Cobalt und/oder Nickel können die Katalysatoren Metalle wie Kupfer oder Chrom, z.B. bis zu 20 Gew.-% auf den Metallgehalt enthalten. Die Katalysatoren können auf Träger, z.B. auf Kieselgel, Magnesiumsilikat, Aluminiumphosphat, Borphosphat, Aluminiumoxid oder Aktivkohle aufgebracht sein. Der Metallgehalt der Trägerkatalysatoren beträgt zweckmäßig 5 bis 80 Gew.-%, bezogen auf die gesamte Katalysatormasse.

Andere geeignete Katalysatoren sind solche, die durch Calcinieren von Verbindungen der Formel I $[Mg_aNi(II)_bCo(II)_cAl_2]CO_3(OH)_{16} \times 4\ H_2O$, in der a für ganze oder Dezimalzahlen von 0 bis 4 steht und b und c für ganze Zahlen oder Dezimalzahlen von 0 bis 6 stehen, mit der Maßgabe, daß $2 \times (a + b + c) = 12$ ist, bei Temperaturen von 200 bis 600°C und anschließende Reduktion mit Wasserstoff bei erhöhter Temperatur hergestellt werden. Bevorzugt geht man von folgenden der Formel I entsprechenden Verbindungen aus:

$Ni_5Al_2(OH)_{16}CO_3 \cdot 4 H_2O$,

$Ni_5MgAl_2(OH)_{16}CO_3 \cdot 4 H_2O$,

$Co_6Al_2(OH)_{16}CO_3 \cdot 4 H_2O$,

$Co_5MgAl_2(OH)_{16}CO_3 \cdot 4 H_2O$.

Die Verbindungen der Formel I erhält man beispielsweise wie folgt: Nickel, Aluminium, Cobalt und Magnesium werden in Form ihrer wasserlöslichen Salze wie Chloride, Sulfate oder vorzugsweise Nitrate gemeinsam in Wasser gelöst und zwar in einem Mengenverhältnis, das der gewünschten Zusammensetzung des Katalysators möglichst nahekommt und in seiner Stöchiometrie der Formel I gehorcht.

Die Metallsalzlösung soll insgesamt etwa 0,5 bis 3 molar, vorzugsweise 1,0 bis 2 molar an Metallionen sein. Sie wird auf Temperaturen von 50 bis 100°C, vorzugsweise 80 bis 100°C erhitzt und innerhalb von 0,5 bis 10, vorzugsweise 1 bis 3 Minuten mit einer äquivalenten Menge oder bevorzugt einem geringen Überschuß, einer auf 50 bis 100°C, vorzugsweise 80 bis 100°C erhitzten 1 bis 3, vorzugsweise 1,5 bis 2,5 molaren Lösungen eines Alkalibicarbonats vereinigt. Vorteilhaft arbeitet man mit einem Überschuß an Alkalibicarbonat, der bis zu 20 Gew.-%, vorzugsweise 0,5 bis 3 Gew.-%, bezogen auf die theoretische Menge des Bicarbonats beträgt. Nach der Zugabe der Metallsalzlösung, nach Vereinigung der beiden Lösungen wird zweckmäßig noch 10 bis 30, vorzugsweise 15 bis 20 Minuten gerührt und dann der entstandene Niederschlag abfiltriert, mit Wasser gewaschen und bei einer Temperatur von 50 bis 200°C, vorzugsweise 100 bis 160°C getrocknet. Die Alkalibicarbonate kommen insbesondere Natriumbicarbonat in Betracht. Es ist aber auch möglich, Ammoniumbicarbonat bei der Fällung zu verwenden. Desgleichen können auch Mischungen der genannten Bicarbonate eingesetzt werden. Außerdem ist es möglich, die Fällung der Metallionen mit Lösungen von Alkalicarbonaten z,B, Natriumcarbonat und/oder Kaliumcarbonat vorzunehmen, wenn man während des Fällens Kohlendioxid in die vorgelegte Alkalicarbonatlösung einleitet, was im Endeffekt auch auf eine Fällung mit Bicarbonat hinausläuft.

Vorteilhaft verwendet man beim Calcinieren Temperaturen von 250 bis 400°C, z.B. für eine Dauer von 5 bis 40, insbesondere 15 bis 30 Stunden an. Vor der eigentlichen Verwendung des Katalysators wird dieser mit Wasserstoff vorteilhaft bei einer Temperatur von 180 bis 500°C, vorzugsweise 250 bis 450°C, z.B. innerhalb von 5 bis 100 Stunden, vorteilhaft 10 bis 25 Stunden reduziert.

Bei der Hydrierung hält man vorteilhaft eine Verweilzeit von 1 bis 120 Minuten ein. Ferner hat sich eine Belastung von 0,2 bis 2 kg 5-Formylvaleriansäure je Liter Katalysator und Stunde bewährt.

Die Umsetzung kann diskontinuierlich, z.B. in einem Druckgefäß oder kontinuierlich in einer Reaktionszone mit fest angeordneten Katalysatoren in Sumpf- oder Rieselfahrweise durchgeführt werden. Es hat sich besonders bewährt, eine Rückvermischung während der Umsetzung zu vermeiden.

In einer dritten Stufe (Stufe c) wird die in Stufe b erhaltene 6-Aminocapronsäure zu Caprolactam cyclisiert. Man verwendet die in Stufe b anfallende Lösung von 6-Aminocapronsäure. Hierzu wird – falls die Hydrierung in Suspensionsfahrweise durchgeführt wurde – zunächst der Katalysator abgetrennt. Dies kann z.B. durch Filtrieren oder Zentrifugieren geschehen. Aus der dann anfallenden ammoniakalischen Lösung der Aminocapronsäure in Wasser wird der Ammoniak vor der Cyclisierung entfernt. Dies kann beispielsweise durch Abdestillieren des Ammoniaks aus der Aminocapronsäure-Lösung erfolgen. Gechieht dies nicht, so werden bei der Cyclisierung niedrigere Caprolactam-Ausbeuten als in Anwesenheit von Ammoniak erzielt. Vorzugsweise hält man in der Ausgangslösung einen Gehalt an Ammoniak von < 1 Gew.-%, insbesondere < 0,5 Gew.-%, ein.

Für die Cyclisierung werden vorteilhaft Lösungen verwendet, die 1 bis 20 Gew.-%, insbesondere 2 bis 10 Gew.-% 6-Aminocapronsäure enthalten. Die Umsetzung wird bei Temperaturen von 150 bis 370°C, insbesondere 250 bis 330°C, im allgemeinen unter dem sich einstellenden Druck des Reaktionsgemisches z.B. von 5 bis 400 bar diskontinuierlich oder kontinuierlich, z.B. in einem Rohrreaktor, durchgeführt. Die mittleren Verweilzeiten betragen hierbei 0,2 bis 2 Stunden.

Die Abtrennung des gebildeten Caprolactams wird bevorzugt extraktiv, z.B. mit Extraktionsmitteln wie Methylenchlorid, Chloroform, Cyclohexan, Toluol, Benzol oder Trichlorethylen durchgeführt.

Das erfindungsgemäße Verfahren sei an folgenden Beispielen veranschaulicht:

Beispiel 1

36 g 5-Formylvaleriansäuremethylester, 90 g Wasser und 2 g Kationenaustauscher aus vernetztem Polystyrol mit Sulfonsäuregruppen wurden 8,5 Stunden unter Rückfluß erhitzt, wobei gebildetes Methanol ständig abdestilliert wurde. Nach fraktionierender Vakuumdestillation wurden 24 g 5-Formylvaleriansäure erhalten (74% d. Th).

Beispiel 2

5-Formylvaleriansäuremethylester (5-FVSE; 5–10 ml/h) und Wasser (50 bis 100 ml/h) wurden kontinuierlich in Rieselfahrweise über zwei beheizbare Doppelmantelstahlrohrreaktoren (Innentemperatur 60 bis 100°C) gepumpt, die mit einem Kationenaustauscher aus vernetztem Polystyrol mit Sulfonsäuregruppen gefüllt waren (Reaktor I: 100 ml Ionenaustauscher, ohne Kreislauf; Reaktor II; 50 ml Ionenaustauscher, 0 bis 30 l/h, Kreislauf). In den Austrägen wurden folgende Mengen 5-Formylvaleriansäure (5-FVS) und 5-GVSE gaschromatographisch bestimmt:

| No | Temp. (°C). | Kreisl.RII (1/h) | 5-FVS (Gew.-%) | 5-FVSE (Gew.-%) | Ausbeute (% d. Th.) | Selekt. % |
|------|------|------|------|------|------|------|
| 4.1 | 60 | 15 | 10,39 | 1,89 | 78 | 89 |
| 4.2 | 80 | 15 | 6,36 | 0,59 | 78 | 84 |
| 4.3 | 100 | 15 | 4,00 | 0,38 | 53 | 56 |

Ein Teil des Reaktionsaustrages (Reaktionstemperatur 60°C) wurde im Vakuum fraktionierend destilliert.

Hierbei wurden 75% d. Th. 5-FVS vom Sdp. 102 bis 123°/l mbar erhalten.

Die Reaktionsausträge wurden mti Cyclohexan extrahiert.

In den wäßrigen Phasen wurden nach der Extraktion folgende Mengen 5-FVS und 5-FVSE gaschromatographisch bestimmt:

| No. | 5-FVS (Gew.-%) | 5-FVSE (Gew.-%) |
|------|------|------|
| 4.1 | 10,28 | 0,21 |
| 4.1 | 6,23 | 0,16 |
| 4.3 | 3,97 | 0,01 |

Beispiel 3

100 ml einer 14,8 gew.-%igen 5-Formylvaleriansäurelösung in Wasser wurden bei 70 bis 130°C/150 bar Wasserstoff zu einer Suspension des Katalysators in wäßrig, ammoniakalischer Lösung in einen 300 ml-Stahlrührautoklaven gepumpt und anschließend 2 Stunden bei Temperatur gerührt.

Die Bestimmung der Aminocapronsäure im Reaktionsaustrag erfolgte durch quantitative HPLC-Chromatographie.

| No. | Temp. (°C) | Katalysator | Ausbeute 6-ACS (% d. Th.) |
|------|------|------|------|
| 6.1 | 70 | 2 g Raney-Nickel | 57 |
| 6.2 | 110 | 2 g Raney-Cobalt | 57 |
| 6.3 | 130 | 2 g Raney-Nickel | 57 |

Vergleichsbeispiel

10 g trimere 5-Formylvaleriansäure, 60 g 12,5%iger wäßriger Ammoniak und 2 g Raney-Nickel wurden 5 Stunden bei 110°C/150 bar Wasserstoff in einen 300 ml-Stahlrührautoklaven gerührt.

Im Reaktionsaustrag wurden durch quantitative HPLC-Chromatographie 31% d. Th. 6-Aminocapronsäure bestimmt.

Beispiel 4

a) 5-Formylvaleriansäure durch Verseifung von 5-Formylvaleriansäuremethylester 5-Formylvaleriansäuremethylester (5-FVSE; 5 bis 10 ml/h) und Wasser (50 bis 100 ml/h) wurden kontinuierlich über zwei beheizbare Doppelmantelstahlrohrreaktoren (Innentemperatur 60°C) gepumpt, die mit einem stark sauren Ionenaustauscher gefüllt waren. (Reaktor I: ohne Kreislauf; Reaktor II: 15 l/h Kreislauf.)

Im Austrag wurden folgende Mengen 5-Formylvaleriansäure (5-FVS) und 5-FVSE gaschromatographisch bestimmt:

| 5-FVS | 5-FVSE | Ausbeute | Selektivität |
|--------|--------|----------|--------------|
| (Gew.-%) | (Gew.-%) | (% d. Th.) | (%) |
| 7,03 | 0,80 | 78 | 85 |

Der Reaktionsaustrag wurde mit Cyclohexan extrahiert. In der wäßrigen Phase wurden nah der Extraktion folgende Mengen 5-FVS und 5-FVSE gaschromatographisch bestimmt:

| 5-FVS (Gew.-%) | 5-FVSE (Gew.-%) |
|----------------|-----------------|
| 6,23 | 0,04 |

b) 6- Aminocapronsäure durch aminierende Hydrierung von 5-Formylvaleriansäure
100 ml 5-Formylvaleriansäurelösung in Wasser (6,23 Gew.-% 5-FVS; Reaktionsaustrag aus a) nach der Extraktion unumgesetzten Esters mit Cyclohexan) wurden bei 110°C/150 bar Wasserstoff zu einer Suspension von 10 g Ru-Zr-Katalysator (0,5 Gew.-% Ru und 1 Gew.-% Zr auf Aluminiumoxid) in 60 g 12,5%igem wäßrigen Ammoniak in einem 300 ml-Stahlrührautoklaven gepumpt und anschließend 2 Stunden bei 110°C gerührt. Nach dem Abkühlen wurde der Katalysator abfiltriert. Die Bestimmung der Aminocapronsäure im Reaktionsaustrag erfolgte durch quantitative HPLC-Chromatographie und ergab eine Ausbeute von 77% d. Th.

c) Caprolactam durch Cyclisierung von 6-Aminocapronsäure
Am Rotationsverdampfer wurde aus dem Reaktionsaustrag aus b) ein Ammoniak/Wasser-Gemisch abgezogen, so daß eine ammoniakfreie, 4,3 gew.-%ige 6-Aminocapronsäure-Lösung in Wasser zurückblieb. Pro Stunde wurden 46,5 ml dieser Lösung bei 300°C durch einen Rohrreaktor (Volumen 46,5 ml) gepumpt. Der nach 19 Stunden Reaktionszeit angefallene Reaktionsaustrag (886 g) wurde mit Chloroform extrahiert. Nach dem Abdampfen des Chloroforms der vereinigten Extrakte wurden 31,1 g Caprolactam (95% d. Th.) erhalten.

**Patentansprüche**

1. Verfahren zur Herstellung von ε-Caprolactam, dadurch gekennzeichnet, daß man
a) 5-Formylvaleriansäure-$C_1$ bis $C_4$-Alkylester mit 1 bis 200 Mol Wasser je Mol 5-Formylvaleriansäure-$C_1$ bis $C_4$-Alkylester in Gegenwart von stark sauren Kationenaustauschern bei einer Temperatur von 50 bis 120°C umsetzt,
b) die so erhaltene 5-Formylvaleriansäure in Form einer wäßrigen Lösung mit 2 bis 50 Mol Ammoniak und 1 bis 10 Mol Wasserstoff je Mol 5-Formylvaleriansäure in Gegenwart von Kobalt-, Nickel- oder Edelmetallkatalysatoren der VIII Gruppe bei einer Temperatur von 50 bis 150°C und unter einem Druck von 10 bis 400 bar umsetzt, und
c) die so erhaltene Lösung von 6-Aminocapronsäure nach Abtrennung des Ammoniaks auf Temperaturen von 150 bis 370°C erhitzt und das gebildete Caprolactam abtrennt.

2. Verfahren nach Anspruch 1, dadurch gekennzeichnet, daß man 50 bis 150 Mol Wasser je Mol 5-Formylvaleriansäureester verwendet.

3. Verfahren nach den Ansprüchen 1 und 2, dadurch gekennzeichnet, daß man 5-Formylvaleriansäureester mit Wasser durch 2 hintereinandergeschaltete Reaktionszonen, die mit stark sauren Kationenaustauschern beschickt sind, leitet, wobei in der zweiten Reaktionszone das Reaktionsgemisch im Kreis geführt wird und die entnommene wäßrige Lösung mit Kohlenwasserstoffen extrahiert wird.

4. Verfahren nach den Ansprüchen 1 bis 3, dadurch gekennzeichnet, daß man die aus der Stufe a erhaltene wäßrige Lösung von 5-Formylvaleriansäure in der Stufe b einsetzt.

5. Verfahren nach den Ansprüchen 1 bis 4, dadurch gekennzeichnet, daß man je Mol 5-Formylvaleriansäure 4 bis 30 mol Ammoniak verwendet.

6. Verfahren nach den Ansprüchen 1 bis 5, dadurch gekennzeichnet, daß man in der Stufe c eine Lösung von 6-Aminocapronsäure mit einem Gehalt von Ammoniak von < 1 Gew.-% einsetzt.

**Claims**

1. A process for the preparation of ε-caprolactam, wherein
a) a $C_1$-$C_4$-alkyl 5 formylvalerate is reacted with from 1 to 200 moles of water per mole of $C_1$-$C_4$-alkyl 5 formylvalerate in the presence of a highly acidic cation exchanger at from 50 to 120°C,
b) the 5-formylvaleric acid thus obtained is reacted in the form of an aqueous solution with from 2 to 50 moles of ammonia and from 1 to 10 moles of hydrogen per mole of 5-formylvaleric acid in the presence of a cobalt, nickel or noble metal catalyst of group VIII at from 50 to 150°C and at from 10 to 400 bar, and

c) after the ammonia has been separated off, the resulting solution of 6-aminocaproic acid is heated to 150-370°C and the caprolactam formed is isolated.

2. A process as claimed in claim 1, wherein from 50 to 150 moles of water are used per mole of 5-formylvalerate.

3. A process as claimed in claim 1 and 2, wherein a 5-formylvalerate is passed with water through two reaction zones which are connected in series and contain strongly acidic cation exchangers, the reaction mixture being circulated in the second reaction zone and the aqueous solution removed being extracted with a hydrocarbon.

4. A process as claimed in any of claims 1 to 3, wherein the aqueous 5-formylvaleric acid solution obtained from stage a is used in stage b.

5. A process as claimed in any of claims 1 to 4, wherein from 4 to 30 moles of ammonia are used per mole of 5-formylvaleric acid.

6. A process as claimed in any of claims 1 to 5, wherein a solution of 6-aminocaproic acid containing < 1% by weight of ammonia is used in stage c.

## Revendications

1. Procédé de préparation d'ε-caprolactame, caractérisé en ce que

a) on fait réagir un ester alkylique en $C_1$–$C_4$ de l'acide 5-formylvalérique avec 1 à 200 moles d'eau, par mole d'ester alkylique en $C_1$–$C_4$ d'acide 5-formylvalérique, en présence d'échangeurs de cations fortement acides, à une température de 50 à 120°C,

b) on fait réagir l'acide 5-formylvalérique ainsi obtenu sous forme de solution aqueuse avec 2 à 50 moles d'ammoniac et 1 à 10 moles d'hydrogène par mole d'acide 5-formylvalérique en présence de catalyseurs de cobalt, nickel et métaux précieux du groupe VIII, à une température de 50 à 150°C et sous une pression de 10 à 400 bars, et

c) on chauffe la solution ainsi obtenue d'acide 6-aminocaproïque après enlèvement de l'ammoniac à des températures de 150 à 370°C et on sépare le caprolactame formé.

2. Procédé selon la revendication 1, caractérisé en ce qu'on utilise 50 à 150 moles d'eau par mole d'ester d'acide 5-formylvalérique.

3. Procédé selon les revendications 1 ou 2, caractérisé en ce qu'on fait passer l'ester d'acide 5-formylvalérique avec l'eau dans deux zones de réaction situées l'une derrière l'autre, qui sont chargées d'échangeurs de cations fortement acides, de sorte que le mélange réactionnel est recyclé dans la deuxième zone de réaction et la solution aqueuse prélevée est extraite avec des hydrocarbures.

4. Procédé selon l'une des revendications 1 à 3, caractérisé en ce que la solution aqueuse d'acide 5-formylvalérique obtenue dans l'étape a est introduite dans l'étape b.

5. Procédé selon l'une des revendications 1 à 4, caractérisé en ce qu'on utilise de 4 à 30 moles d'ammoniac par mole d'acide 5-formylvalérique.

6. Procédé selon l'une des revendications 2 à 5, caractérisé en ce qu'on introduit dans l'étape une solution d'acide 6-aminocaproïque avec une teneur en ammoniac de moins de 1% en poids.